# EUROPEAN PATENT SPECIFICATION

(11) **EP 2 093 980 B1**
(45) Date of publication and mention of the grant of the patent: **01.05.2019**
(21) Application number: 09250421.6
(22) Date of filing: 18.02.2009
(51) Int. Cl.: G08B 5/22, G06F 19/00, G16H 40/63

(54) **User station for healthcare communication system**
Benutzerstation für ein Kommunikationssystem des Gesundheitswesens
Poste d'utilisateur pour système de communication de soins de santé

(30) Priority: 22.02.2008 US 66882 P
(43) Date of publication of application: 26.08.2009
(73) Proprietor: Hill-Rom Services, Inc., Batesville IN 47006 (US)
(72) Inventor: Schuman, Richard Joseph, Cary, NC 27511 (US); Collins, Williams F. Jr.,, Columbus, IN 47203 (US); Roehl, Erik Edward, Apex, NC 27539 (US)
(74) Representative: Findlay, Alice Rosemary

(56) References cited:
- EP-A2- 1 623 666
- US-A1- 2003 093 300

## Description

The present disclosure relates generally to healthcare communication systems such as patient-nurse communication systems, and more particularly to user stations usable in connection with such systems.

Healthcare communication systems such as patient-nurse communication systems or "nurse call" systems enable communication among members of a nursing staff and other persons dispersed throughout a healthcare facility. Such systems generally provide information about the current status or condition of patients in the facility, and enable voice communication between patients and staff members through a telecommunications infrastructure.

One example of a known nurse call system that includes a user station is Hill-Rom's COMLINX® system. In the COMLINX® system, a "master station" is provided, which is configured to oversee the operation of the system for a specific territory within a facility, such as a nursing unit or units or the entire facility. The master station communicates call information to audio stations that are positioned at various locations throughout the monitored territory. Types of audio stations include patient stations (also called "room stations"), which are located in patient rooms, and staff stations, which are located in designated staff areas. The prior art audio stations have more limited functionality than a master station. For instance, prior art audio stations generally provide for viewing call information in a limited fashion, placing calls, answering unanswered calls, and canceling calls originating from the location in which the audio station is installed.

There is still a need for advanced healthcare communication system capabilities directed to improving nursing staff and overall hospital efficiency. Additional or improved features that are directed to reducing the risk of adverse patient conditions occurring in the facility are also needed. However, as cost is often a concern to these facilities, advancements that can be achieved while containing or reducing the costs of implementing, maintaining and operating these systems are desired.

US2003/0093300 discloses a healthcare communication system which can include a patient terminal having a keyboard unit with message keys for transmitting different ones of one or more programmed messages, and alphanumeric keys for composing messages. The patient terminal can be communicatively linked to a patient terminal display for presenting the programmed messages and composed messages. Each of the message keys can include identifying indicia associated with a programmed message transmitted upon selection of the message key. The patient terminal keyboard also can include one or more destination keys for specifying one of multiple destination addresses within the healthcare communication system for delivering individual ones of the messages.

EP 1 623 666 discloses a system that monitors various conditions of a plurality of hospital beds located in different rooms of a healthcare facility. Alternatively or additionally, other types of equipment may be monitored by the system. Various configurations of network interface units that are coupleable to or integrated into a hospital bed are also disclosed. The system receives data from the hospital beds and/or other equipment and initiates a communication to a wireless communication device of at least one designated caregiver in response to the received data being indicative of an alarm condition.

The invention provides a patient-nurse communication system according to claims 1-3.

The invention will now be further described by way of example with reference to the accompanying drawings, in which:
Fig. 1 is a simplified schematic showing a logical architecture for a patient-nurse communication system in communication with other components of the system;
Fig. 2 is a simplified schematic showing physical components of a patient-nurse communication system showing connectivity to other services and systems;
Fig. 3 is a simplified diagrammatic view of an exemplary implementation of a patient-nurse communication system in a patient care facility;
Fig. 4 is a front elevational view of a user station including a plurality of connector ports, a speaker, a microphone, a plurality of buttons and a plurality of visual indicators;
Fig. 5 is a front elevational view of a user station similar to the embodiment of Fig. 4, including removable side portions, each of which includes a plurality of connector ports; and a "code" or emergency call button;
Fig. 6 is a perspective view of a user station similar to the embodiments of Figs. 4 and 5, including a visual display portion and a multifunctional user control;
Fig. 7 is a front elevational view of a user station similar to the embodiments of Figs. 4 and 5, showing a plurality of connectors connected to the connector ports;
Fig. 8 is as front elevational view of a user station including a graphical display supported by the front face, a plurality of user controls, a plurality of visual indicators, and a speaker;
Fig. 9 is a perspective view of a user station including a front face, a graphical touch display supported by the front face, a microphone, first and second laterally spaced sides, each including a speaker grille; a top side, a bottom side longitudinally spaced from the top side, and a code call lever adjacent the top side;
Fig. 10 is a front elevational view of a graphical touch display for a user station, including a tabular listing of calls, a scroll bar, a plurality of functional tabs, a plurality of buttons, and a plurality of icons;
Fig. 11 is a perspective view of a user station including an enlarged graphical touch display supported by a housing having a front face, the display including a plurality of programmable windows, icons, buttons, text fonts and text lists; a top side, a bottom side longitudinally spaced from the top side, a first side, a second side laterally spaced from the first side, a code call lever coupled to the top side, and a microphone supported by the second side;
Fig. 12 is a perspective view of a user station including a housing having a front face, first and second laterally spaced sides, a top side and a bottom side longitudinally spaced from the top side, and a back side spaced from the front face, an enlarged graphical touch display with enhanced resolution supported by the front face, a microphone flush with the front face, a speaker grill located on at least one of the laterally spaced sides, a desk mount supporting the housing, and a telephone handset adjacent the front face;
Fig. 13 is a front elevational view of a user station similar to the embodiment of Fig. 12, configured to be mounted to a wall, headwall, wall units, and other substantially vertical structures;
Fig. 14 is a graphical touch display for a user station, including a plurality of programmable windows, icons, buttons, text fonts and text lists; showing a first window containing a call list and a second window containing a staff list, each of the windows being scrollable, and a plurality of functional tabs and other controls that are touch-activatable;
Fig. 15 is an exploded front perspective view of a user station including a first housing portion having a front face, a plurality of buttons and visual indicators supported by the front face, a code call lever positioned along the top side of the first housing portion, a speaker grill in a least one of the laterally spaced sides of the first housing portion, a second housing portion including a back face and a pair of laterally spaced sides, and a pair of speakers, each mounted to one of the laterally spaced sides;
Fig. 16 is an exploded rear perspective view of a portion of the user station of Fig. 15, showing a universal mounting plate and connector slots in a rear portion of the housing;
Fig. 17 is a rear exploded perspective view of the first housing portion of the user station of Fig. 15, showing a code call lever and a coupler to pivotably couple the code lever to the housing;
Fig. 18 is another rear exploded perspective view of the first housing portion of the user station of Fig. 15, showing a plurality of touch switches supported by the first housing portion, an infrared sensor and mounting apparatus for securing the infrared sensor in the housing, the code call lever supported by the top side and pivotably coupled to one of the laterally spaced sides of the first housing portion, and speaker grills on each of the laterally spaced sides of the first housing portion;
Fig. 19 is a front exploded perspective view of another embodiment of a user station, similar to the embodiment of Fig. 9, having a first housing portion configured to support a code call lever, an infrared sensor and having an aperture sized to receive a graphical touch display, and a second housing portion configured to support the graphical touch display and a pair of laterally spaced speakers;
Fig. 20 is a rear exploded perspective view of the user station of Fig. 19, a mounting apparatus, and a mounting box configurable to mount the user station to a vertical structure, the user station including a plurality of slots configured to receive fingers of the mounting apparatus, the user station including a plurality of connector ports configurable to connect a variety of computing devices and computer accessories to the user station;
Fig. 21 is a front perspective view of the user station of Fig. 19 mounted to the mounting box;
Fig. 22 is a front exploded perspective view of the mounting apparatus of Fig. 20, having a top side and a bottom side longitudinally spaced from the top side, a plurality of mounting fingers arranged laterally along the top and bottom sides, and a pair of laterally elongated mounting slots provided in the top and bottom sides; and showing a mounting box including a plurality of mounting ports on a top side and a plurality of mounting ports on a bottom side longitudinally spaced from the top side;
Fig. 23 is a front perspective view of another embodiment of a user station, similar to the embodiment of Fig. 13, including a housing having a front face, a rear face, a top side, a bottom side longitudinally spaced from the top side, a first side and a second side laterally spaced from the first side, the front face including an aperture sized to receive an enlarged graphical touch display, a code call lever positioned above the top side, and a speaker grille located in at least one of the laterally spaced sides;
Fig. 24 is a rear exploded view of a portion of the user station of Fig. 23, including a first housing portion, a second housing portion insertable in the first housing portion, and a touchscreen display assembly mountable to the first housing portion, the second housing portion having a pair of speakers mounted to laterally spaced sides thereof;
Fig. 25 is a second rear exploded view of another portion of the user station of Fig. 23, showing components mounted to touchscreen display and a rear housing portion;

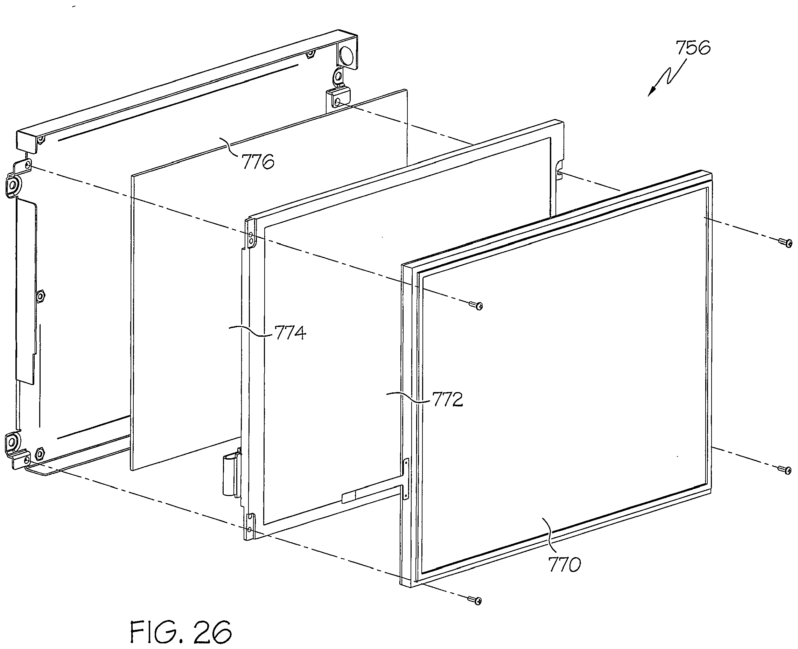

Fig. 26 is a front exploded perspective view of the touchscreen display assembly of Figs. 24-25;
Fig. 27 is a rear exploded perspective view of a back housing portion for a desk mounted user station similar to Fig. 12, including a rear cover, a microphone, a pivot connector and a mounting stand; and
Fig. 28 is another rear exploded perspective view of the back housing portion of Fig. 27, showing the back cover, mounting stand, a back portion of the user station housing mountable to the back cover, the back portion of the user station including a plurality of connector ports, and a handset cradle assembly mountable to the back cover.

Aspects of the present invention are described with reference to certain illustrative embodiments shown in the accompanying drawings and described herein.

In general, a healthcare communication system includes one or more staff or nursing computers or computing devices, which may be referred to as stations or consoles. The stations or consoles, in cooperation with various computers, networks, and supporting equipment and services, enable nurses and other staff to receive, view, manage, and route, output or respond to electrical and wireless signals from a variety of communication, call, monitoring, detecting and/or signaling devices. Some communication, call, monitoring, detecting and/or signaling devices are operated by patients, staff, or visitors. Others are activated by the occurrence of an event or condition detected by signal receivers, patient monitoring equipment or hospital beds located throughout a healthcare facility. When the system receives a signal from a communication, call, monitoring, detecting and/or signaling device, one or more indicator assemblies may be activated to alert hospital staff of the condition or event being signaled by the communication, call, monitoring, detecting and/or signaling device.

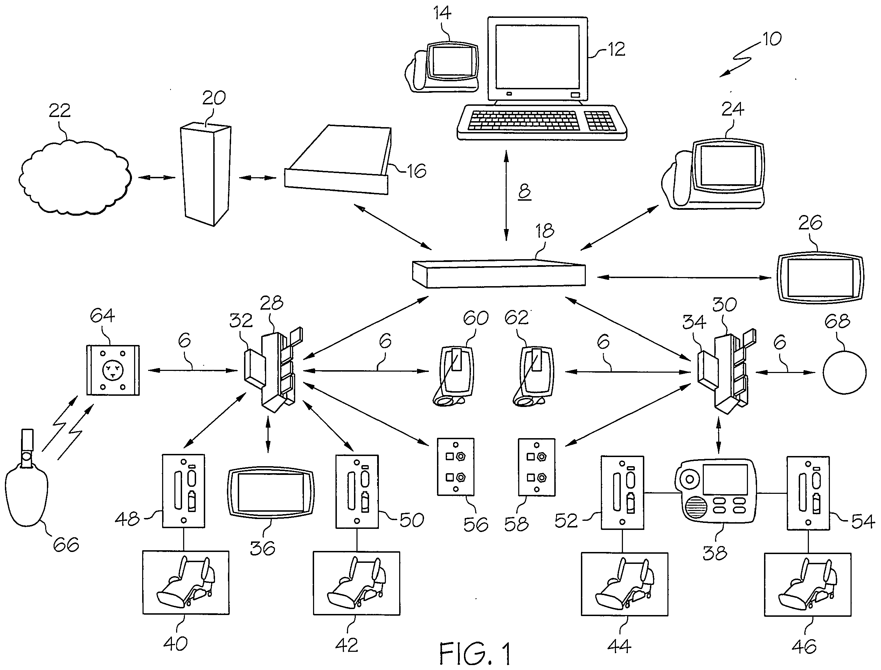

One embodiment of a patient-nurse communication system 10 is diagrammatically illustrated in Fig. 1. System 10 includes a primary user console or station 12, and one or more secondary user consoles or stations 14, 24, 26, 36, 38 which are configured to be operated by nurses or other staff. Primary station 12 enables nurses or staff to monitor activity and communicate with patients and other staff within the facility or portion of the facility monitored by the system. Primary station 12 is a computer or computing device that has a display screen, voice communication capabilities, and one or more input devices (such as a keyboard, touch screen, mouse, switch, button, knob, or the like) configured to control the operation of the patient-nurse communication system. Voice communication capabilities are provided by an integrated microphone and speaker and/or a telephone handset.

Primary console or station 12,14 is configured to enable a nurse or other staff to place calls, cancel calls, monitor the location of other staff members, process calls and alerts and route or relay calls or alerts to and from other consoles or other components of the system. Primary console 12 may further be configured to enable an authorized user to update the status of calls, alerts, monitored persons and/or monitored devices or equipment, and enable or disable calls or alerts. Primary station 14 is configured to be desk-mounted but could also be wall-mounted.

Secondary user consoles or stations 24, 26, 36, 38 have similar components and provide similar but often more limited capabilities than the primary console 12, 14. For example, primary console 12 may include a larger display screen, a graphical user interface configured for data entry, monitoring, and analysis, a network interface (e.g., for TCP/IP connectivity), and/or a telephone handset. However, different configurations of secondary consoles 24, 26, 36, 38 exist that may or may not have a graphical display or telephone handset, or may have limited network connectivity.

For example, console 24 has structural components that are similar to console 14 but generally does not have all the same functional capabilities as console 14 because console 24 is a secondary console. Console 24 may be configured to display only a subset of the information that is available at console 14 (i.e., console 24 may be configured to display only calls pertaining to a particular grouping of patient rooms assigned to a specific nurse, while console 14 is configured to display all call information for all rooms in a nursing unit, group of units or entire facility). Consoles 26, 36 have similar structural components and functional capabilities as console 24 but do not have a telephone handset. Console 38 is a scaled-down and potentially lower cost version of console 24, and as such has more limited graphic capabilities and restricted network connectivity.

Notwithstanding the above description, secondary consoles 24, 26, 36, 38 may have all of the components and functional capabilities as primary console. For example, a console or station may be a primary console for one nursing unit, zone or portion of a facility and also be configured as a secondary console for another unit, zone or portion of the facility. In this way, information for multiple units, zones or portions of a facility may be monitored from one station or console.

Consoles 12, 14, 24, 36, 38 are connected either directly or indirectly (i.e., through an electrical assembly, such as an input-output board) to a switch 18. In the illustrated embodiment, switch 18 is a Power over Ethernet (POE) switch, however, other suitable types of switches may be used, as will be understood by those skilled in the art. Switch 18 and electrical assemblies or input-output boards 32, 34 provide connectivity to a variety of call, communication, monitoring, detecting and/or signaling devices 40, 42, 44, 46, 48, 50, 52, 54, 56, 58, 60, 62, 64, 66, 68 to receive call and/or alert signals therefrom. Switch 18 may also be configured to provide electrical power to remote devices, as is the case with POE switches.

In general, "console" or "station" is used herein to refer to a computer or computing device configured to provide an interface to the system for a user, such as a nurse, staff member, patient, or visitor. As such, these equipment generally include at least one output device, such as a visual display or speaker, to notify or communicate calls and/or other information to the user. Stations or consoles may also include at least one input device, such as a touchscreen, keypad or keyboard, microphone, telephone handset, push button, switch, dial, lever, or the like, to enable the user to place and/or respond to the calls or other information. Stations or consoles also include circuitry to connect them to the system 10. Stations or consoles include embodiments that may be desk- or table-mounted, as well as embodiments that may be mounted to a wall, headwall, column, bed, siderail, or other structure.

Input/output boards 32, 34 are circuit board assemblies that provide computing processing and wiring for a patient location in the healthcare facility, such as a patient room. Among other things, the electrical assemblies or I/O boards operate to convert device-specific protocols from a variety of devices, which may be installed in patient rooms, to a single network protocol suitable for communication over a network. For example, I/O boards 32, 34 convert serial links to primary and secondary consoles, remote locating receiver or bed interface unit room bus protocols, and serial to dome light protocols, on the one side, to XML-over-TCP/IP on the other side. In the illustrated embodiments, each I/O board 32, 34 includes a multimedia microprocessor with built-in multimedia capability, such as the Freescale IMX 27. Input-output boards 32, 34 may also include one or more POE ports to enable devices to connect directly to the board instead of connecting to the system through a switch.

Indicator assemblies 28, 30 are coupled to electrical assemblies or input-output boards 32, 34 and receive control signals therefrom to activate a visual or audible notification, or a combination of visual and audible notifications, at the indicator assembly.

In general, primary console 12 is in communication with electrical assemblies or input-output boards 32, 34 through a computer network 8 and switch 18. Secondary consoles 14, 24, 26, 36, 38 are in communication with primary console 12 over network 8 through a switch 18 and may thereby receive information and commands from primary console 12. In the illustrated embodiment, network 8 is a TCP/IP network running an XML data protocol configured to enable communication among a number of devices and/or systems usable by the healthcare facility.

Call, communication, monitoring, detecting and/or signaling devices include, for example: beds 40, 42, 44, 46 (such as Hill-Rom TotalCare® or VersaCare® beds), which are linked to system 10 via bed interface units 48, 50, audio bed station connectors (ASBCs) 52, 54, or similar bed connector devices; patient monitors and other medical or clinical devices or equipment (such as therapy equipment, heart rate or respiration monitoring devices, and the like), which are linked to system 10 via connectors 56, 58; call cords 60, 62; wireless (i.e. infrared or radio frequency) location tracking receivers or "remote location receivers" 64 and related location tracking badges or tags 66, and smoke alarm 68. Some call, communication, monitoring, detecting and/or signaling devices, such as remote receiver 64, cords 60, 62, smoke alarm 68 and bed interface units 48, 50, are coupled directly to I/O boards 32, 34 by communication links 6. Other devices are coupled to I/O boards 32, 34 indirectly through consoles or stations, such as ASBCs 52, 54, which connect beds 44, 46 to station 38. In the illustrated embodiment, links 6 are RS485 connections.

For ease of description, this disclosure may use "incoming call" or "call" to refer to one or more calls, messages, communications or signals sent from a call, communication, detecting, monitoring, and/or signaling device to system 10, and may use "outgoing notification", or "notification" to refer to one or more calls, messages, communications, alarm signals, alert signals or other indications or annunciations that are configured to notify or otherwise direct the attention of a nurse or other staff member of or associated with the facility to an incoming call. Further, this disclosure may use "call device" to refer individually or collectively to such call, communication, detecting, monitoring, and/or signaling devices.

As shown in Fig. 1, switch 18 links various components of system 10 to a primary station 12, 14. Primary station 12, 14, alone or in combination with one or more other server computers and/or computing devices, hosts and executes software and services needed to operate system 10. Primary station computer 14 is configured to process control messages generated by system 10 and send them to the appropriate destination or endpoint, such as a secondary console, I/O board, or other electrical assembly. As such, server 14 includes a soft telephony switch and related componentry.

Server 14 is configured to operate and manage many of the primary nurse call functions of system 10, such as receiving and managing messages from various connected devices, synchronizing devices that come online, controlling placement and canceling of calls, answering of calls, generating of notifications or alerts, acknowledging and canceling of notifications and alerts, managing location information for staff and devices, activating and deactivating staff, managing staff-patient assignments, assigning and managing roles and responsibilities to staff and devices, and managing patient information and patient discharges and transfers.

Switch 18 may also link system 10 to an "enterprise" server 16. Enterprise server 16 may be configured to enable system 10 to interface with systems or services that are considered "external" or "optional" to system 10. For example, server 16 may be coupled to a telecommunications server 20, which acts as a gateway to a facility's telecommunications infrastructure 22. Infrastructure 22 generally includes a network that is configured to facilitate communication among a variety of telecommunication devices, including analog and digital devices, fixed telephones and mobile or cellular devices, personal data assistants (PDAs), pagers and the like. For example, infrastructure 22 may include a public switched telephone network (PSTN) or private branch exchange (PBX) or the like.

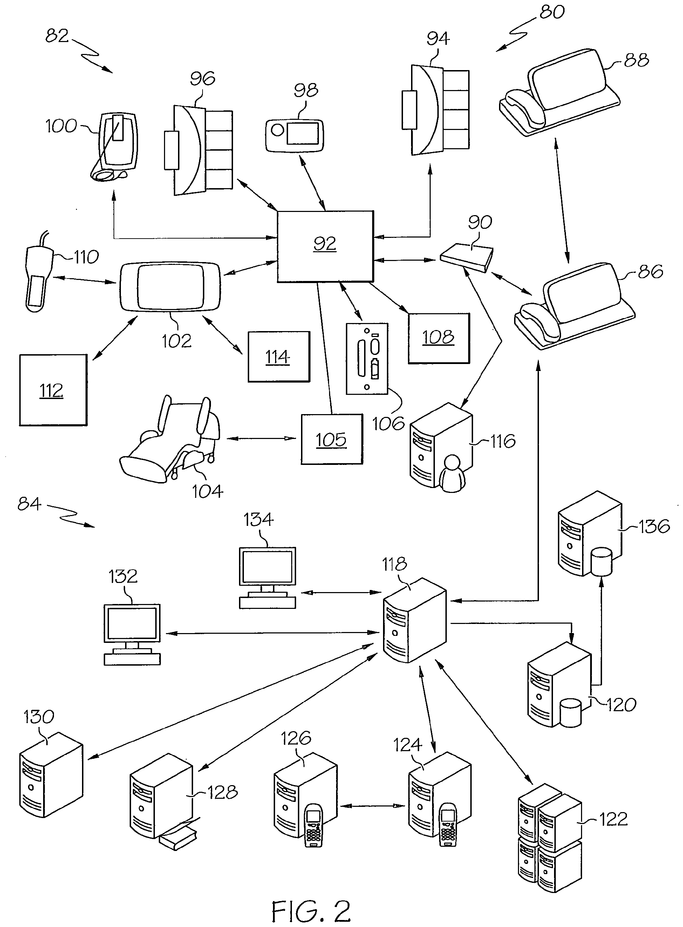

Fig. 2 illustrates connectivity among components of an embodiment of a healthcare communication system 80 including a nurse call system 82 and a plurality of other services and/or systems 84. Nurse call system 82 includes a primary console 86 operably coupled to a switch 90, and a secondary console or station 88 logically coupled to primary console 86 and physically coupled to switch 90. Secondary console 88 is configured to display information about a nursing unit or unit(s) for which it is not the primary console.

Switch 90 is operably coupled to I/O board 92 and server 116. I/O board 92 is configured to receive incoming calls from a variety of devices connected thereto, including but not limited to indicator assemblies 94, 96, secondary console 98, call cord or switch 100, secondary console 102, bed 104, bed interface unit 106, remote locating receiver 108, and pillow speaker 110. In general, these devices are connected to I/O board 92 by an RS 485 link. Additional devices, such as bed connector 112 and call cord 114, may be coupled to or integrated with a secondary console such as console 102 and thereby connected to system 80. One embodiment of an electrical assembly or I/O board is IBM Part No. 43T2063.

An interface 105 is operable to connect bed 104 to I/O board 92. In the illustrated embodiment, interface 105 is a 37 pin connector facing outward that a bed plugs into. On the other side of the plate 105, inside the back box, wires are connected to each pin of the 37 pin connector that could be run to other devices that the bed controls, such as lighting controller, TV, radio, and nurse call patient stations. It may be used in place of a bed interface unit or ASBC.

Server 116 is a VOIP server configured to translate system operations and communications to the corresponding messages that then control endpoint devices, such as nurse or staff stations, consoles or room input/output boards. As such, server 116 includes a soft telephony switch and other associated componentry. Server 116 may also provide integration with the hospital telecommunications structure (e.g., PBX or other voice communication system). In the illustrated embodiment, server 116 is a Windows server running 3CX.

Primary console 86 may optionally be coupled to a second server 118 by a network 115, such as a TCP/IP network. Server 118 may also be coupled to switch 90. Server 118 is similar to enterprise server 16 described above.

Other services and systems of system 84 are in communication with network 115 through server 118. Such other services or systems may include a database server 120, one or more third party servers 122, a first wireless communications server 124 for managing communications to and from wireless telecommunications devices, a second wireless communications server 126 for handling communications to and from wireless badges for locating and tracking of staff members, a user authentication server 128 for managing user accounts, passwords, and user authorization; a third party product integration server 130, which facilitates integration with third party or legacy products or services; a hospital administrative client 132 for conducting administrative tasks relating to patients and staff, such as adding patients and assigning staff to patients; and a status or reports server 134 for managing displays and reports of calls and notifications for one or more locations in the facility.

While the term "server" is used herein, it will be understood by those skilled in the art that the functionality represented or performed by these elements may comprise software programs or services that may be resident and/or executable by any computer, device or equipment in the system or more than one computer, device or equipment in the network.

In the illustrated embodiment, server 124 is configured to provide communication and configuration for wireless devices using Emergin Wireless Office; server 126 is configured to provide communication and configuration for wireless Vocera devices; server 130 is configured to interface with a Hill-Rom NaviCare system to receive and process alerts therefrom; and server 134 is configured to operate an "electronic status board," which displays locations within the facility and current information about them, such as active calls, bed status information, staff located in the location, and staff assigned to the location.

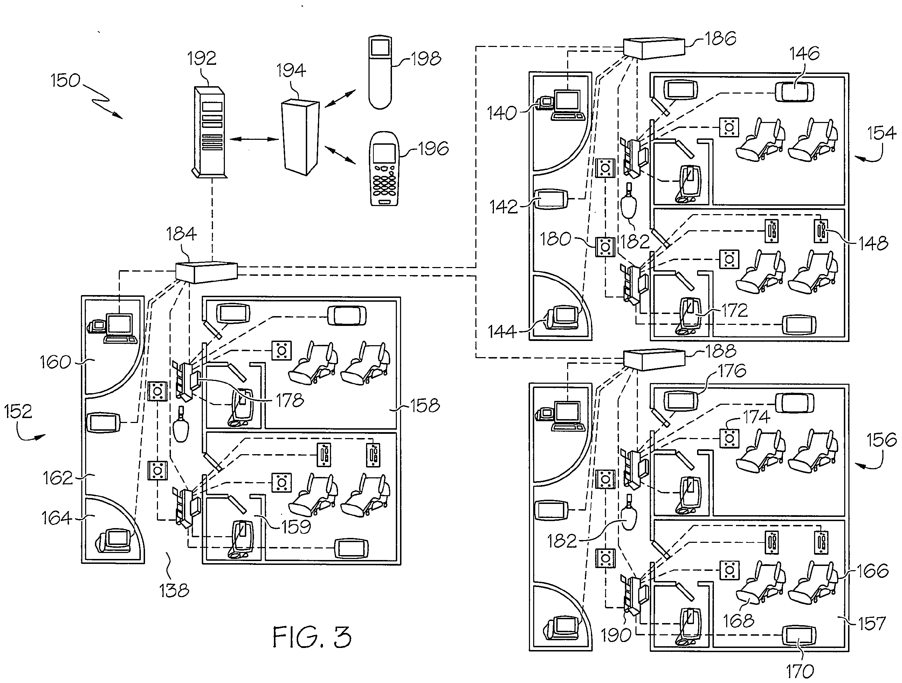

Fig. 3 diagrammatically shows an illustrative implementation in a facility of a healthcare communication system 150 including many of the components described above. The illustrated facility has a plurality of nursing units or zones 152, 154, 156, each of which has one or more patient rooms or locations 158, hallways or common areas 138, and staff locations 160, 164. Each patient room 158 has a bathroom or washroom 159.

A number of call monitoring and/or communication or signaling devices are located throughout the facility, including primary consoles 140, secondary consoles 142, 144, 146, 170, 176, bed interface units 148 and beds 166, 168, toilet, bath and/or shower switches 172, wireless locating receivers 174, 180, and wireless locating transmitter badges 182. In the illustrated configuration, each nursing unit 152, 154, 156 includes a primary console 140, and each patient room includes at least one secondary console 146 and at least one switch 172 located in the bath/washroom 148.

An indicator assembly 190 is mounted in the hallway 138 outside of each patient room 158. Indicator assemblies 190 may be mounted either to a wall or ceiling, above the door to the room or in another suitable location indicative of the patient room with which the indicator assembly is associated. An electrical assembly or I/O board 178 is also associated with each patient room and may be mounted adjacent to each indicator assembly. Additional details regarding indicator assemblies of the type referred to herein may be found in the European Patent Application entitled INDICATOR ASSEMBLY FOR HEALTHCARE COMMUNICATION SYSTEM, filed on the same day as the present application.

Secondary consoles 142, 144 may also be located in hallways 138 and staff locations 160, 164. Locating and tracking receivers 174, 180 are provided in the patient rooms 158, hallways 138 and other locations.

A POE switch 184, 186, 188 is associated with each unit 152, 154, 156 and operably coupled to the devices of its respective unit. System server 192 is coupled to switch 184, which is in turn coupled to switches 186, 188 in the illustrated embodiment. System server 192 is similar to server 118 described above. VOIP server 194 is operably coupled to server 192 and to telecommunications devices 196, 198, substantially as described above.

In operation, when a call or signal is initiated by one of the call initiating devices, executable computer logic processes the call or signal, determines which nurse or staff member to notify of the call, if a notification is necessary, locates the nurse or staff member, and routes an appropriate notification or notifications to one or more output devices associated with the assigned nurse or staff member or within the closest proximity to the assigned nurse or staff member. At the same time, a notification is routed to the output device nearest the location where the call originated. Such computer logic may be located in memory at a primary console, I/O board or at the application server 192.

For example, if a nurse is assigned to units 152 and 156, is currently tending to a patient in room 157 of unit 156, and a patient or piece of monitoring equipment in room 158 issues a call, then system 150 locates the nurse using room receivers 174 and hall receivers 180 and the nurse's badge 182. System 150 then activates the appropriate visual and/or audible notifications at the indicator assembly 178 assigned to the patient room where the call originated. System 150 may activate a visual and/or audible notification at the console 170, nearest the nurse's location, as well. System 150 may cancel or disable one or more of the notifications when the locating receivers detect that the nurse has departed the area or when the nurse enters the room 158 where the call originated.

Additional details describing the structural components, connectivity, functionality, and other operations of the above-described communication systems may be found in European Patent Applications entitled DISTRIBUTED HEALTHCARE COMMUNICATION SYSTEM, and DISTRIBUTED FAULT TOLERANT ARCHITECTURE FOR A HEALTHCARE COMMUNICATION SYSTEM, filed on the same day as the present applications and incorporated herein by reference.

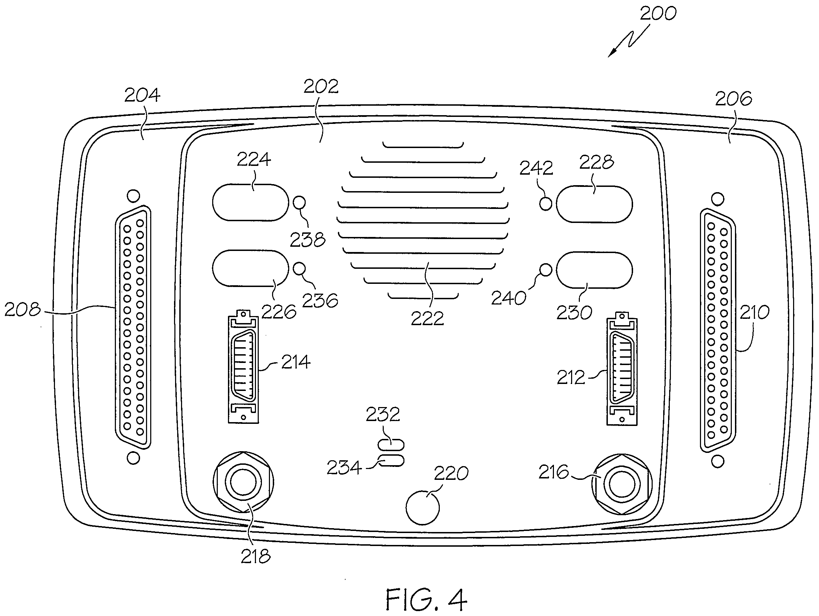

Various embodiments of a user station suitable for use in a healthcare communication system such as described above and in the related applications are shown in Figs. 4-24 and described below.

Fig. 4 is a front elevational view of a user station 200 including a plurality of connector ports 208, 210, 212, 214, 216, 218, a speaker 222, a microphone 220, a plurality of buttons 224, 226, 228, 230 and a plurality of visual indicators 232, 234, 236, 238, 240, 242. All of the aforementioned elements are located on the front face 202, which has a first side face 204 and a second side face 206 located on either side. Connectors 208, 210 are 37-pin connectors suitable for connecting user station 200 to a hospital bed system, such as Hill-Rom's TotalCare® or VersaCare® beds), or to other monitoring or therapy equipment to receive signals therefrom and convey the signals to the healthcare communication system. Connections 212, 214,216, 218 may be used to connect a pillow speaker, call cord, or other equipment or devices from which it may be desirable to receive signals to convey to the healthcare communication system. User station 200 is capable of receiving and outputting voice communications but does not have a graphical user interface.

In general, buttons 224, 226, 228, 230 are user input devices such as membrane switches or other electromechanical buttons, which enable a user to place calls of different types (e.g., normal, code blue, staff call, staff emergency, etc.) and cancel a call previously made from the station. Visual indicators 232, 234, 236, 238, 240, 242 are light emitting diodes (LEDs) that are generally lit when a particular function of the station 200 is active and unlit when the function is not active. For example, LED 232 is lit when a call has been placed, and LED 234 is lit when an audio communication line is open, allowing the user to convey voice communications through the healthcare communication system through user station 200.

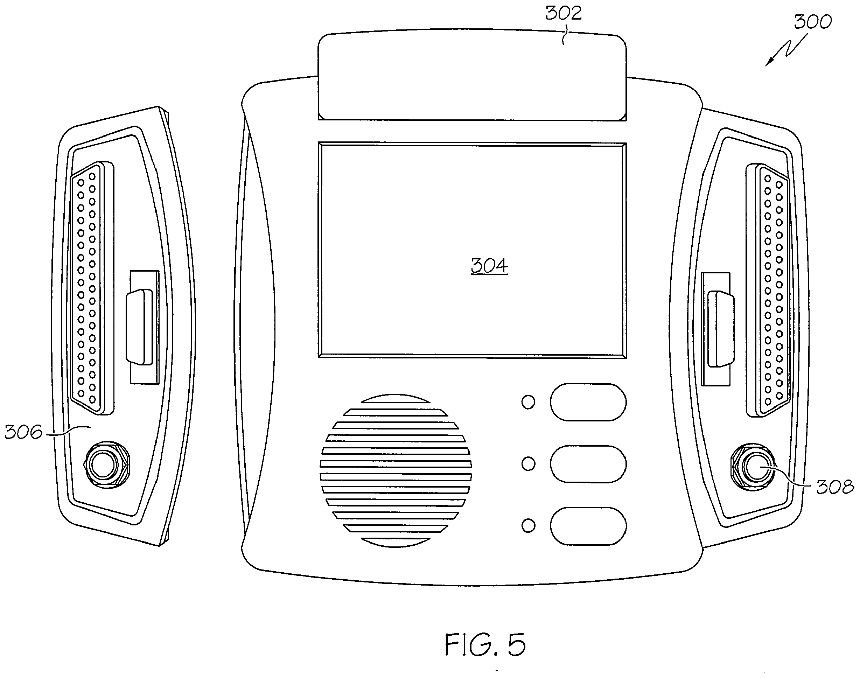

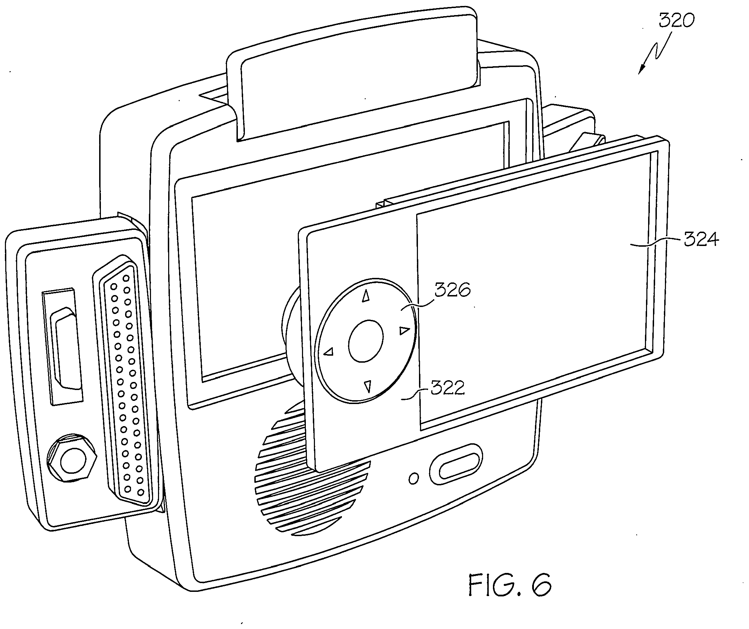

Fig. 5 is a front elevational view of a user station 300, which is similar to the embodiment of Fig. 4, including removable side portions 306, 308, each of which includes a plurality of connector ports; and a "code" or emergency call button 302. However, while embodiment 200 does not have graphical user interface capabilities, embodiment 300 includes an area 304, which may be configured to support a visual display, such as a graphical user interface or LCD display 324, as shown in the embodiment 320 of Fig. 6. Visual indicator assembly 322 of Fig. 6 includes display 324 and user input device 326, which operates similarly to a computer mouse or track ball, to enable a user to select, activate or deactivate options displayed on the user interface 324. The removability of side portions 306, 308 from user station 300 provide adaptability and scalability, so that side portions 306, 308 can be removed from user station 300 in facilities where bed and/or equipment monitoring features are not needed or desired, potentially resulting in a cost savings to the facility.

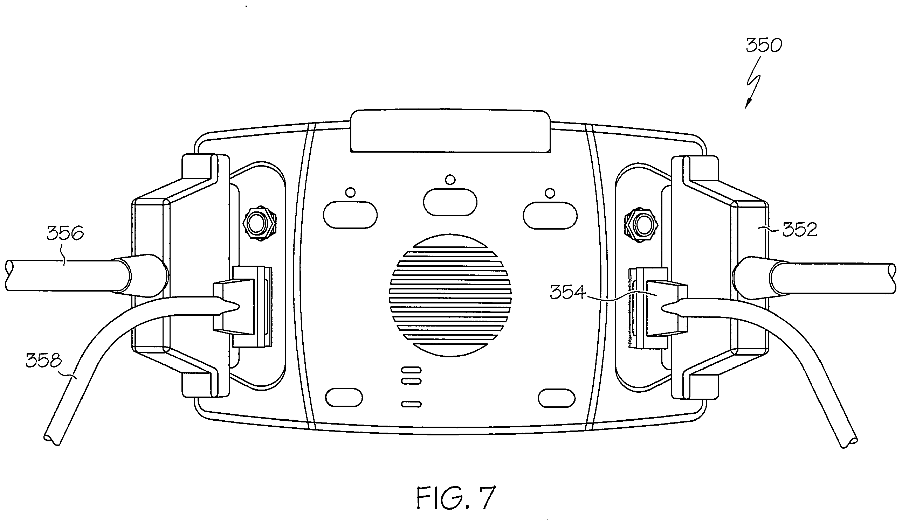

As noted above, Fig. 6 is a perspective view of a user station 320 similar to the embodiments of Figs. 4 and 5, including a visual display portion 324 and a multifunctional user control 326. Fig. 7 is a front elevational view of a user station 350 similar to the embodiments of Figs. 4 and 5, showing a plurality of connectors 352, 354, 356, 358 connected to the connector ports.

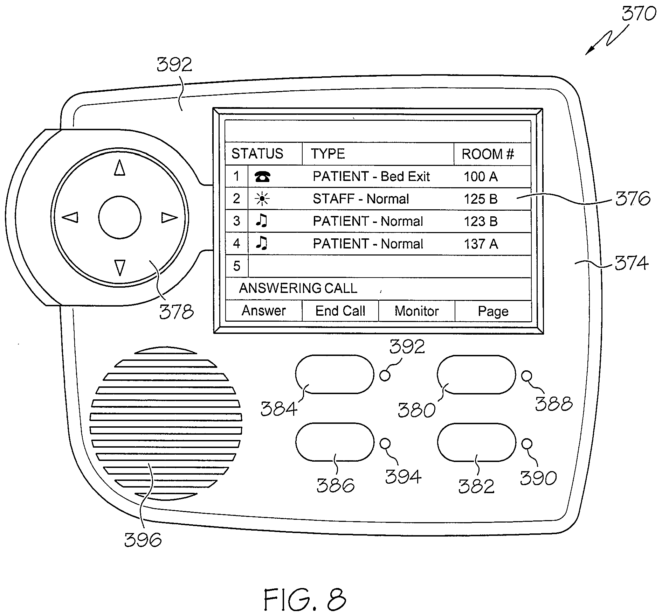

Fig. 8 is as front elevational view of a user station 370 including a housing 374 having a front face 372, a graphical display 376 supported by the front face 372, a plurality of user controls 380, 382, 384, 386 usable to place or cancel calls to the healthcare communication system, a plurality of visual indicators (LEDs) 388, 390, 392, 394, a speaker 396 and a user input device 378. Graphical display 376 is generally an LCD display but does not have touch input capability; hence, user input device 378 is configured to enable a user to scroll through the call list and activate or deactivate the features of the display 376 (such as answer call, end call, monitor calls, and page a staff member), much like a computer mouse or track ball-type device.

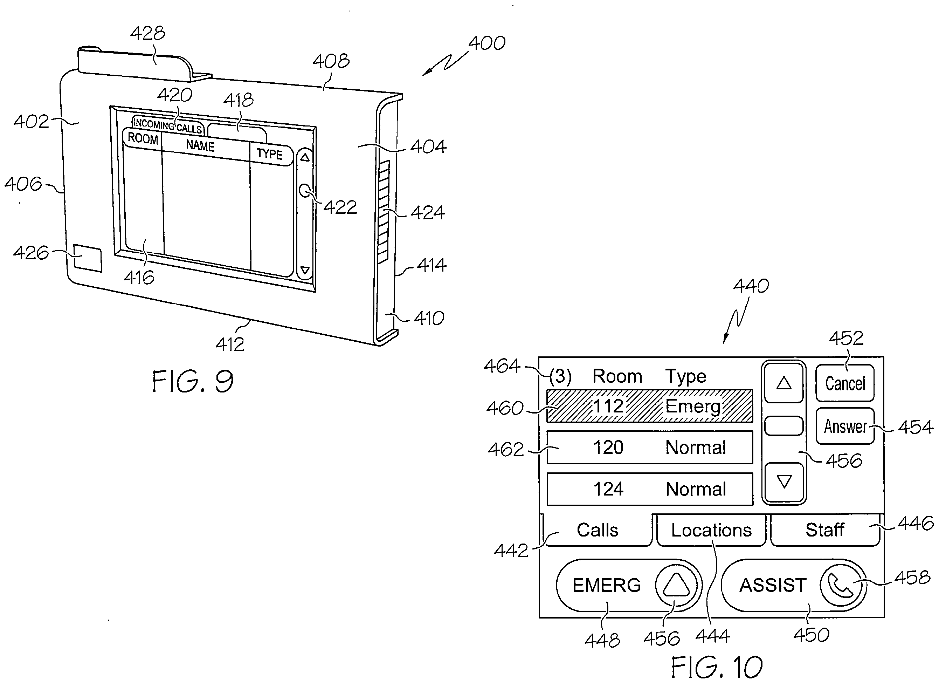

Fig. 9 is a perspective view of a user station 400 having a housing 402 including a front face 404, laterally spaced sides 406, 410, a top side 408 and a bottom side 412 longitudinally spaced from the top side 408. Station 400 includes a graphical touch display 416 supported by the front face 404, and a microphone 426. First and second laterally spaced sides 406, 410 each include a speaker grille 424. A code call lever 428 is provided adjacent the top side 408. Station 400 is typically installable in patient rooms, near the headwalls, for example. As such, it is configured to be mountable to a wall, headwall, architectural wall unit or other substantially vertical structure.

Station 400 may also be used as a staff or duty station to place and receive calls from areas normally reserved for staff members. As such, computer program logic or software is executed to configure station 400 for use by a patient or a staff member. For instance, graphical display 416 will embody more limited functionality when configured for patient use (i.e., it will enable a patient to place calls but not to view the call list or monitor calls). The configuration of front face 404 is such that display 416 and microphone 426 are in substantially the same plane as the front face housing 404, such that the entire front face including these elements is substantially smooth in appearance and structure. The absence of ridges or other nonconformities that may result from the use of pushbuttons, LEDs, and placement of speaker on the front face, enables the front face to be more easily cleaned. The use of touch sensors or actuators on the display 416 eliminates the need for physical buttons and results in a smooth, cleanable surface. The side-mounted speakers make the front face easier to clean as well.

Station 400 includes a printed circuit board assembly including electrical componentry, such as a multimedia microprocessor and other related components, to enable pre-recorded audio files (e.g., .wav files) to be output by the speakers of station 400. The ability to play pre-recorded sound files offers flexibility in designing and recording sounds to meet varying needs of facilities. In one embodiment, station 400 includes two circuit boards, a main board and a daughter board mounted to the main board. The daughter board may be used to house connectors for Ethernet componentry or for other purposes. One embodiment of such a circuit board is IBM Part No. 43T2071.

The circuitry of station 400 also includes IEEE 802.3af compliant components so that station 400 can be powered by Power over Ethernet (PoE) network switches. The circuitry of station 400 also includes componentry that incorporates session initiation protocol (SIP) voice over Internet protocol (VoIP) within the station itself. Station 400 also includes software executable by a processor to enable a user to select the method of contacting another user (such as voice routed to a located staff position, wireless telephone or other wireless device, or text page to a wireless device. Support for PoE reduces the number of cables connected to the station by combining power and network connectivity in one cable. IEEE 802.3af compliance provides flexibility to user a variety of network switches marketed by numerous manufacturers and thereby enhance cost competitiveness. Inherent SIP compliant VoIP provides flexibility to use a variety of private branch exchange (PBX) products marketed by numerous manufacturers and thereby enhance cost competitiveness. These aspects of station 400 may also be incorporated into other embodiments of stations described herein. They are also described in the aforementioned related patent applications. Exemplary embodiments of station 400 are IBM Part No. 43T2071 and IBM Part No. 43T2067 and IBM Part No. 43T1863.

Fig. 10 is a front elevational view of a graphical touch display 440 for a user station, including a tabular listing of calls 460, 462, a touch-activated scrolling mechanism 456, a plurality of touch-activated functional tabs 442, 444, 446, a plurality of touch-activated buttons 448, 450, 452, 454, and a plurality of icons 456, 458. Enhanced graphics capabilities are used to selectively highlight or shade certain areas of the display, for example, the first call 460 in the list is highlighted relative to the other calls in the list, and the first functional tab 442 is set off graphically from the currently inactive tabs 444, 446. Further, icons 456, 458 are set off from the rest of the display by the use of different colors. For example, the emergency icon 456 is red while the assistance icon 458 is yellow.

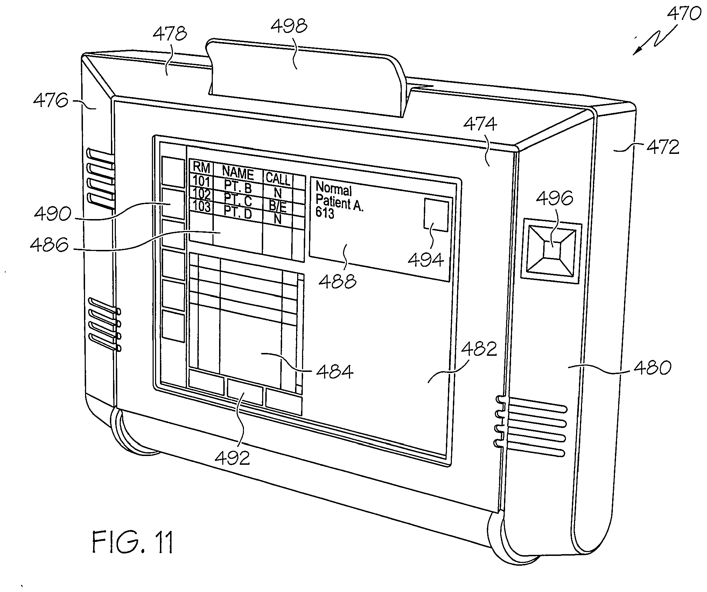

Fig. 11 is a perspective view of a user station 470 including an enlarged graphical touch display 482 supported by a housing 472 having a front face 474, and beveled sides 476, 478, 480. In the illustrated embodiment, the graphical display is about 10 inches in size. The display 482 includes a plurality of programmable windows 484, 486, 488, icons and touch activated buttons 490, 492, 494, text fonts and text lists as shown. Station 470 has a code call lever 498 coupled to the top beveled side 478, thereby not contacting front face 474, and a microphone supported by the beveled side 480, also thereby not in contact with front face 474.

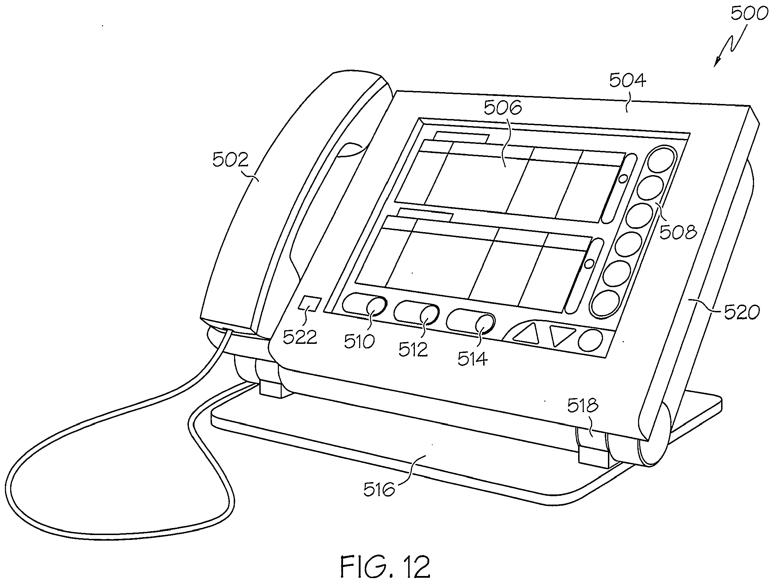

Fig. 12 is a perspective view of a user station 500 including a housing having a front face 504, first and second laterally spaced sides, a top side and a bottom side longitudinally spaced from the top side, and a back side spaced from the front face. An enlarged graphical touch display 506 with enhanced resolution is supported by the front face. A microphone 522 is substantially flush with the front face, as is display 506. A speaker grill 520 is located on at least one of the laterally spaced sides. A desk mount 515 supports the station housing, and a pivot coupler 518 enables pivoting of station 500 relative to desk mount 515. A telephone handset 502 is provided adjacent the front face 504. Display 506 includes touch activated buttons and icons 508, 510, 512, 514, which are set off from each other by selective coloring, shading or highlighting as described above, for ease and efficiency of use, so that a user does not have to take time to carefully search the display for the appropriate button. A Super Video Graphics Array (SVGA) touch display may be used, and a VGA and higher resolution touch display may be used.

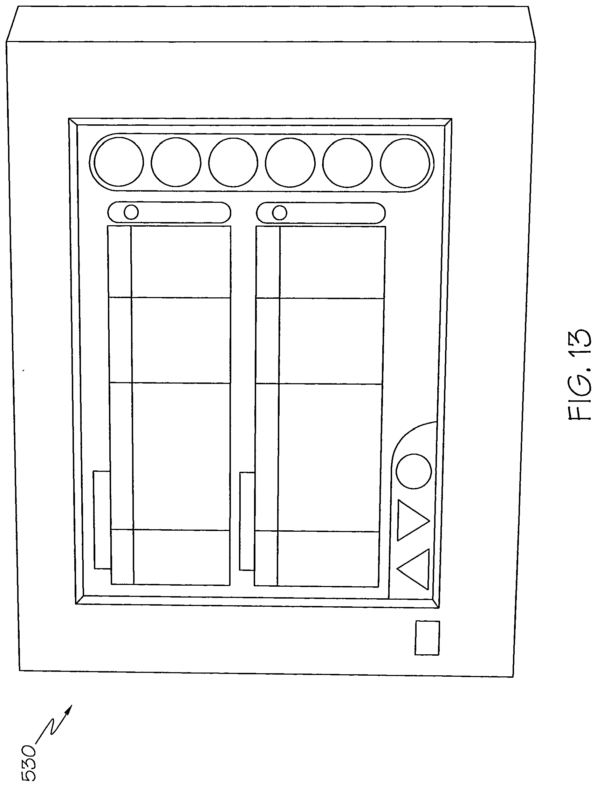

Fig. 13 is a front elevational view of a user station 530, which is similar to the embodiment of Fig. 12, however station 530 is configured to be mounted to a wall, headwall, wall units, and other substantially vertical structures. Exemplary embodiments of user stations 500, 530 are IBM Part No. 43T2058, IBM Part No. 43T1871, and IBM Part No. 43T1866.

Stations 470, 500 may be used as either a staff console, a staff station/annunciator, or a patient station. Computer software is executable to program or configure the graphical display to provide the functional capabilities that are appropriate for the particular selected use. In general, either of stations 470, 500 may be used as the primary user interface for nurses and other staff members to view and answer incoming calls and to communicate with patients or staff. Stations 470, 500 may also be programmed to enable users to view staff location information.

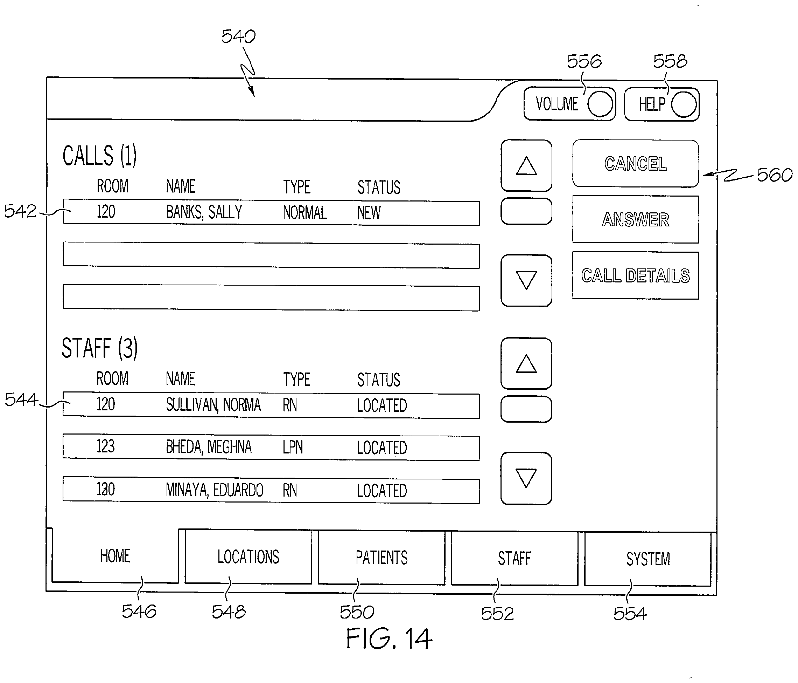

Fig. 14 is a graphical touch display 540 for a user station, including a plurality of programmable windows, icons, buttons, text fonts and text lists. Display 540 includes a first window 542 containing a call list and a second window 544 containing a staff list. The total number of calls and located staff is displayed, as shown. Each of the windows are scrollable by a touch activated control on the display screen. A plurality of functional tabs 546, 548, 550, 552, 554 and other controls are touch-activatable. Relative to display 440, display 540 generally provides additional functionality. For instance, display 540 provides the user with capabilities for monitoring and managing patient information via tab 550 and for managing system information via tab 554, in addition to viewing and managing calls and staff locations. Enhanced graphics provide selective highlighting or shading. For instance, buttons 560 are shown in a muted or faded shade to quickly indicate to the user that those features are currently unavailable. A touch activated volume control 556 is also provided on the display screen 540.

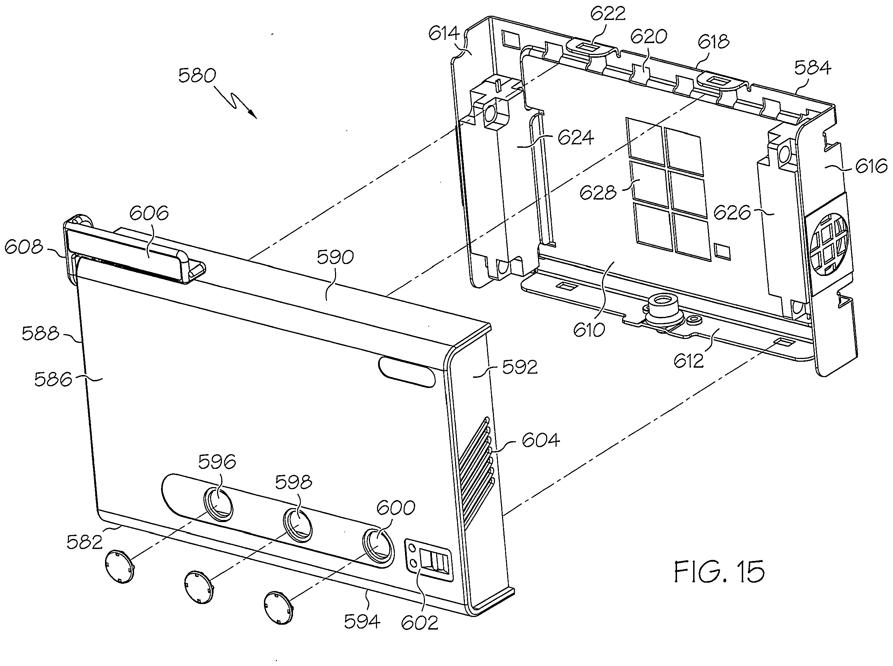

Fig. 15 is an exploded front perspective view of a user station 580, which is similar to stations 400 and 530 but without the graphical display capabilities. Perspective views of an embodiment similar to station 580, showing the front face and sides, and including a microphone and call placed indicator supported by the front face, are shown in Figs. 29-31. A back elevational view of user station 580 showing a plurality of connector ports to connect a variety of devices, equipment and/or services to the user station is shown in Fig. 32.

Exemplary embodiments of station 580 are IBM Part No. 43T2082 and IBM Part No. 43T1862. Station 580 includes a first housing portion 582 having a front face 586, a plurality of apertures 596, 598, 600 proximate bottom side 594 and configured for installation of capacitive touch actuators, a code call lever 606 positioned along the top side 590 of the first housing portion 582, a speaker grill 604 in a least one of the laterally spaced sides 588, 592 of the first housing portion 582, and an infrared receiver 602 configured to receive infrared signals from tags or badges emitting IR signals for locating and tracking of staff and/or equipment throughout the healthcare communication system. Lever 606 is pivotably mounted to side 588 by lever mount 608.. a second housing portion including a back face and a pair of laterally spaced sides, and a pair of speakers, each mounted to one of the laterally spaced sides.

Second housing portion 584 attaches to first housing portion 582 by couplers 622. Second housing portion 584 includes a plurality of mounting slots 620 configured to receive mounting fingers of a universal mounting plate as described below and shown in Fig.15. A pair of speakers 624, 626 are mounted to sides 614, 616 respectively. A plurality of apertures 628 are provided to receive a plurality of mounting ports to connect station 580 to the healthcare communication system, computer networks, other computing devices and accessories, such as a computer mouse, keyboard, camera, external video monitors with touch capability, or the like. Dual side mounted speakers allow sound to be projected out both sides to make it easier to be heard, and also results in a "clean" front face to make the station easier to clean. The capacitive touch actuators also eliminate the need for physical buttons and results in a smoother cleanable surface.

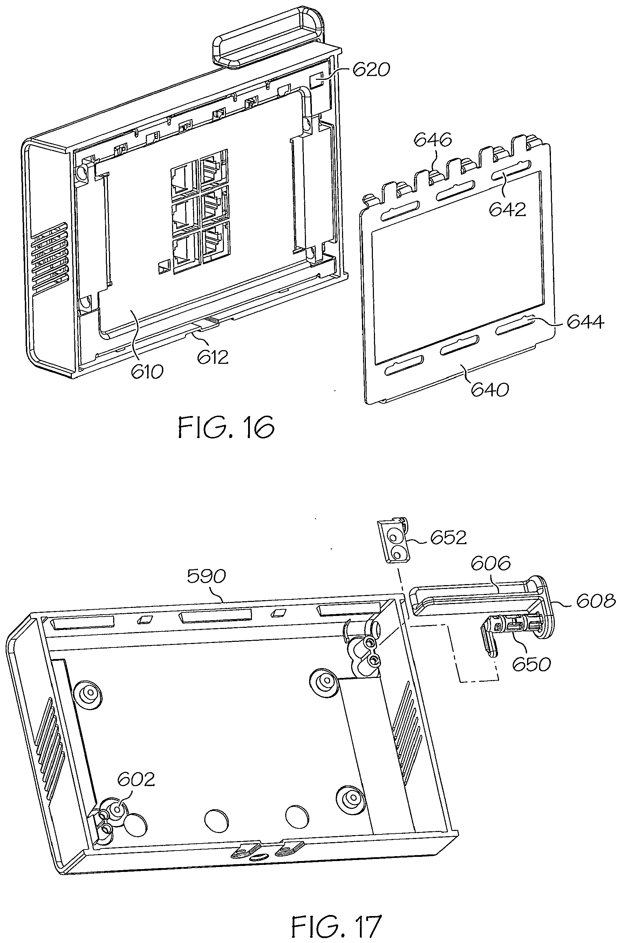

Fig. 16 is an exploded rear perspective view of a portion of the user station 580 of Fig. 15, showing a universal mounting plate 640 and mounting slots 620 in a rear portion of the housing 612, laterally spaced to align with prongs or fingers 646 of mounting plate 640. Mounting plate 640 also includes a pair of longitudinally spaced laterally elongated mounting slot 642, 644.

Mounting plate 640 is configured to mount any of the user stations 400, 530, 580 to a wall outlet box or back box. The flexible mounting design allows stations to be installed onto 2-gang, 3-gang, or 4-gang outlet boxes with continuous, side to side adjustment capability. Flexible mounting may reduce installation costs for a facility, since whatever configuration of back boxes is already installed may be used to mount the stations. The flexible mounting design also overcomes wall construction discrepancies and tolerances, in addition to providing flexibility to use one of several sizes of back boxes. The flexible mounting also enables wall mountable user stations to "nest" with other wall-mountable system components, such as bed connector units (e.g., an Audio Station Bed Connector or ASBC) so that both the station and the other unit can be mounted side by side to one mounting box. The nested mounting configuration enabling one-box mounting of these components may reduce installation costs, since only one back box is required to mount both the user station and the other unit.

Fig. 17 is a rear exploded perspective view of the first housing portion 590 of the user station 580 of Fig. 15, showing a code call lever 606 and a coupler 608, 650, 652 to pivotably couple the code call lever to the housing. The side-mounting configuration of call lever 606 provides the code blue call lever functionality without degrading the appearance of the front face of the station when the call lever option is not included. Thus, the same housing may be used whether or not the station will provide the code call lever. Also, the side mounting removes the lever from the front face, aiding in the cleanability of the front face.

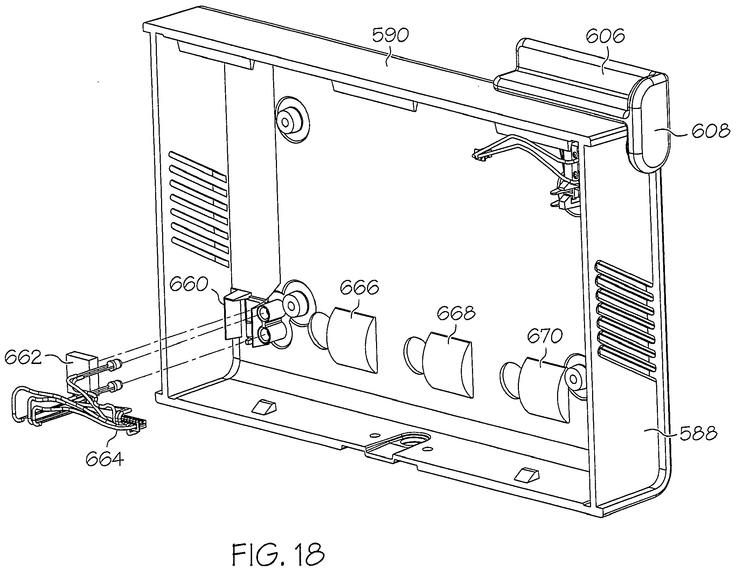

Fig. 18 is another rear exploded perspective view of the first housing portion 590 of the user station 580 of Fig. 15, showing a plurality of touch switches 666, 558, 670 supported by the first housing portion 590, an infrared locating sensor assembly 662, 664 and mounting apparatus 660 for securing the infrared sensor in the housing, the code call lever 606 supported by the top side and pivotably coupled to one of the laterally spaced sides of the first housing portion by coupling portion 608, and speaker grills on each of the laterally spaced sides 588, 592 of the first housing portion.

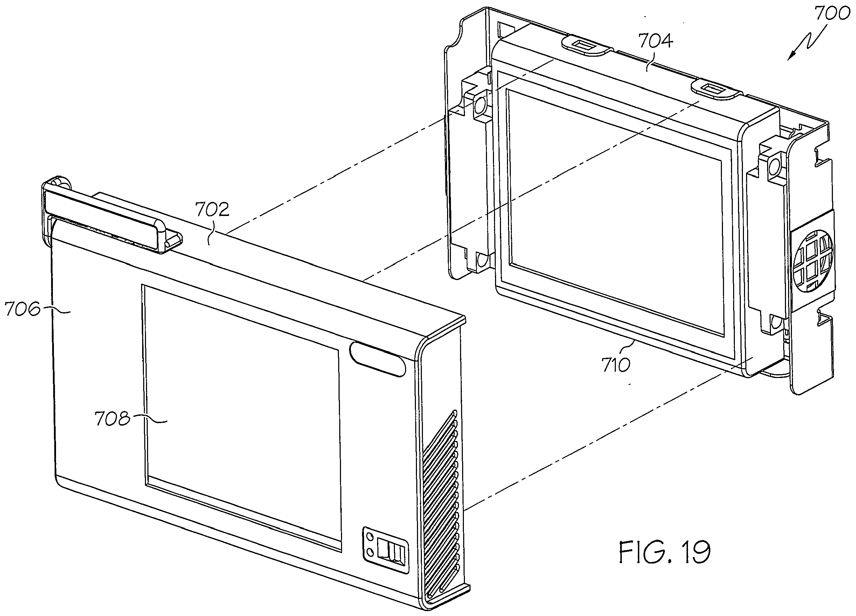

Fig. 19 is a front exploded perspective view of another embodiment 700 of a user station, similar to the embodiment of Fig. 9, having a first housing portion 702 configured to support a code call lever, an infrared sensor and having an aperture 708 sized to receive a graphical touch display 710. A second housing portion 704 is configured to support the graphical touch display 710 and associated circuitry, as well as a pair of laterally spaced speakers as described above.

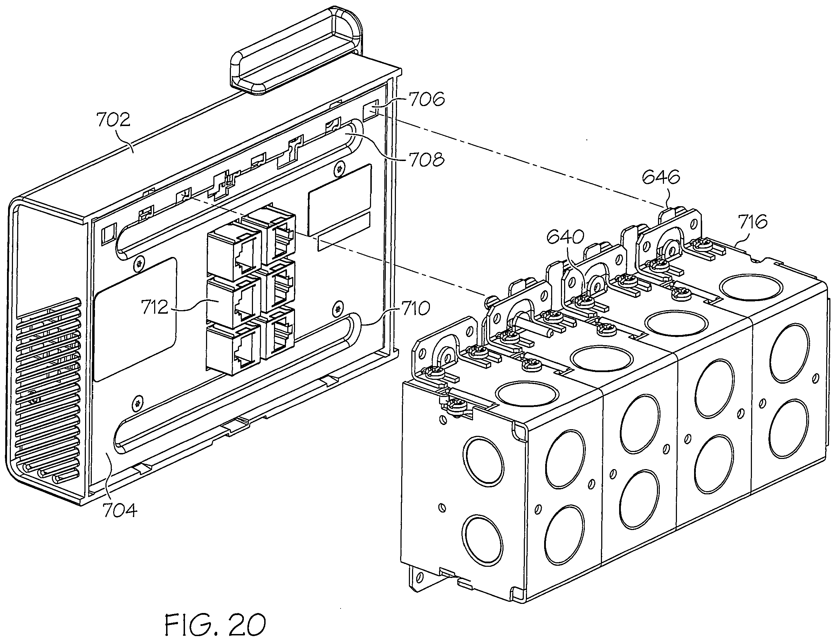

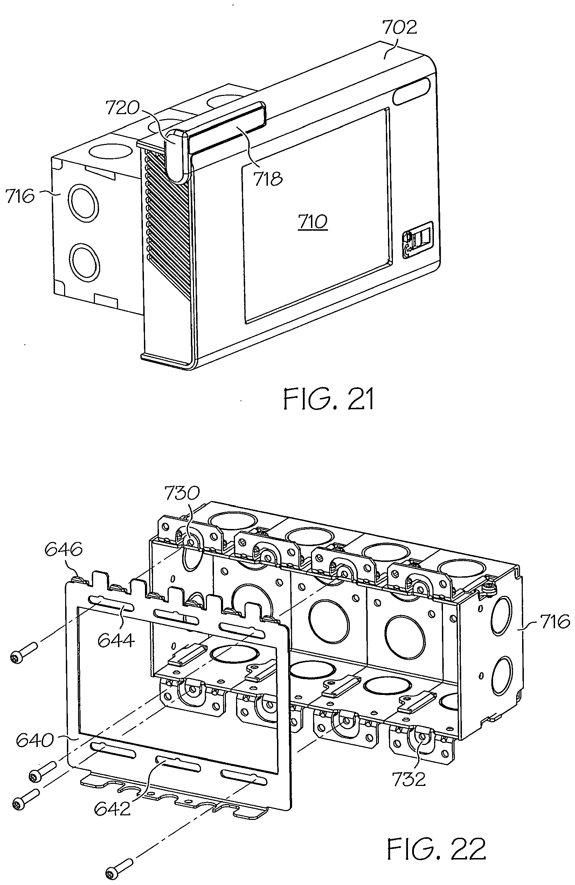

Fig. 20 is a rear exploded perspective view of the user station 700 of Fig. 19, a mounting apparatus 640 including a plurality of fingers 646 configured to engage slots 706 of housing 702 to couple station 700 to a mounting box 716, which is configurable to mount the user station 700 to a vertical structure. User station 700 includes a plurality of connector ports 712 configurable to connect a variety of computing devices and computer accessories to the user station, for example, video and touch adapters to allow the connection of external video monitors and touch capability, USB ports to attach computer accessories, a PoE port, and the like. Fig. 21 is a front perspective view of the user station 700 of Fig. 19 mounted to the mounting box 716. Mounting apparatus 640 is configured to be able to mount a variety of embodiments of user stations to a variety of different mounting boxes.

User station 700 includes a graphical display 710 and optionally, a code call lever 718 mounted to housing 702 by mounting portion 720. Graphical display 710 may be of one of the types described above in connection with the descriptions of stations 400, 530, 470, 500.

Fig. 22 is a front exploded perspective view of the mounting apparatus 640 of Fig. 20, having a top side and a bottom side longitudinally spaced from the top side, a plurality of mounting fingers 646 arranged laterally along the top and bottom sides, and a pair of laterally elongated mounting slots 642, 644 provided in the top and bottom sides as described above. Screws or other fasteners insert into slots 642, 644 of apparatus 640 and mounting ports 730, 732 of mounting box 716, while fingers 646 couple to the user station housing as described above, to couple the station to the box. The mounting box including a plurality of mounting ports 730 on a top side and a plurality of mounting ports 732 on a bottom side longitudinally spaced from the top side.

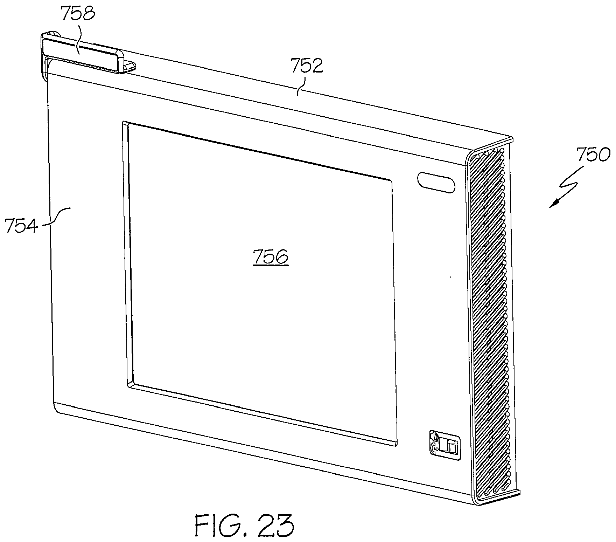

Fig. 23 is a front perspective view of another embodiment of a user station 750, similar to the embodiment of Fig. 13, including a housing having a front face 754, a rear face spaced from the front face, a top side 752, a bottom side longitudinally spaced from the top side, a first side and a second side laterally spaced from the first side to define an interior region. The front face 754 includes an aperture 756 sized to receive an enlarged graphical touch display as described above. Front face 754 optionally supports a code call lever 758 positioned above the top side, and a speaker grille located in at least one of the laterally spaced sides. Station 750 has similar graphical capabilities as stations 470, 500, 530 described above. Relative to station 700, the graphical display of station 750 generally takes up a larger area of the front face.

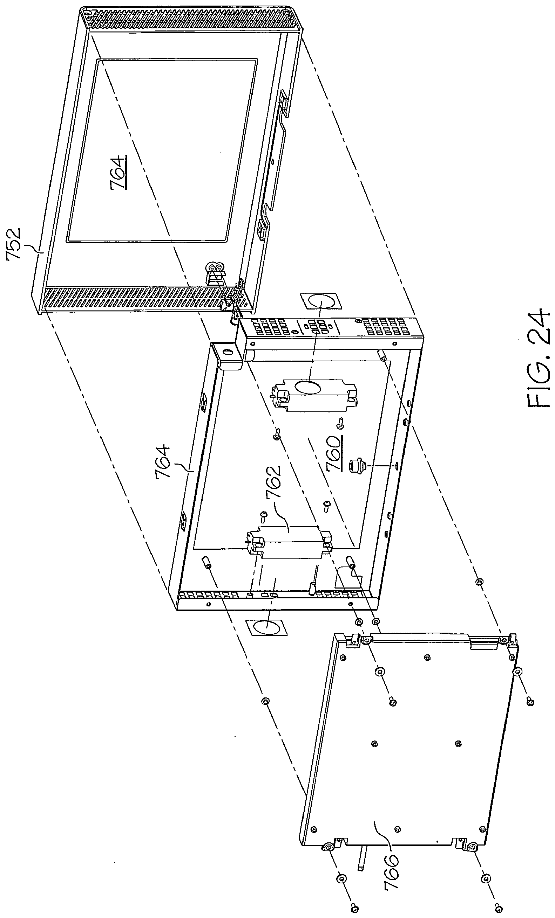

Fig. 24 is a rear exploded view of a portion of the user station 750 of Fig. 23, including first housing portion 752, a second housing portion 764 insertable in the first housing portion 752, and a touchscreen display assembly 766. The second housing portion 764 has a pair of speakers 760, 762 mounted to laterally spaced sides thereof.

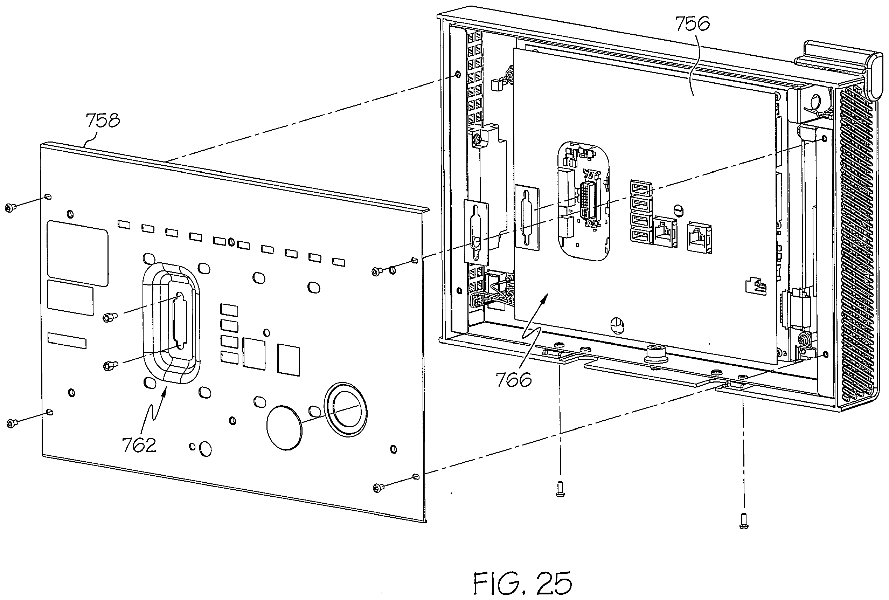

Fig. 25 shows the first housing portion 752 with second portion 764 and touchscreen 766 installed therein. The printed circuit board assembly 776 includes componentry relating to touchscreen 766, as well as other computer circuitry configured to operate the features and functions described herein. A plurality of connectors 766 are provided as described above. Rear cover 758 includes a plurality of apertures 762 of varying sizes to accommodated devices, cabling and wiring connectable to ports 766. Touchscreen assembly 756 includes a touch sensor panel 770, an LCD display 772 (i.e., VGA or SVGA), an insulator 774 and a printed circuit board assembly 776 as described above.

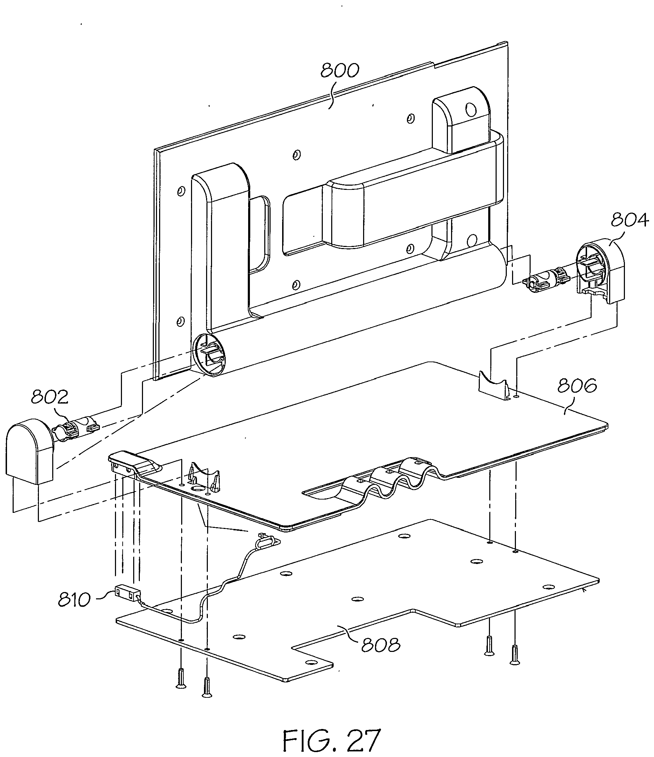

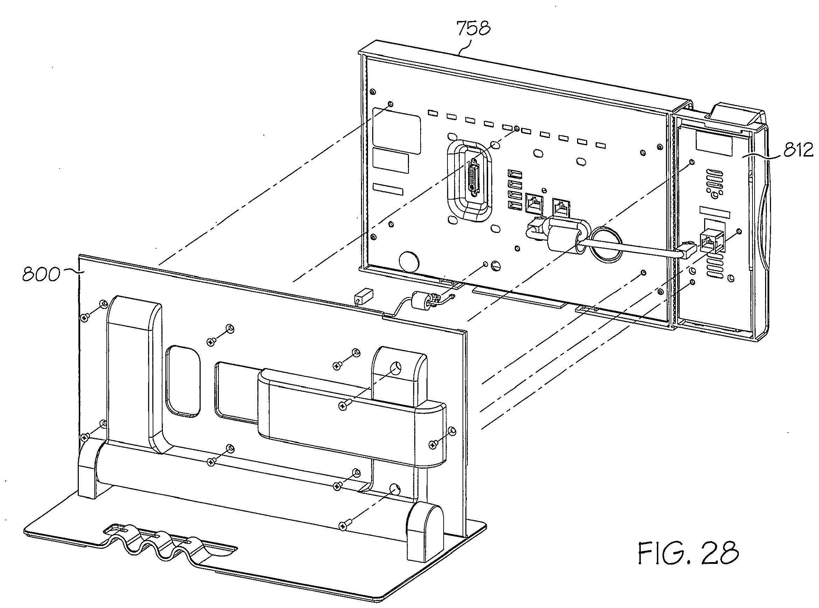

Figs. 27-28 are rear exploded perspective views of a back housing portion 800 for a desk mounted user station similar to Fig. 12, including a rear cover 800, a microphone 810, a pivot connector 802, 804 (such as a friction hinge) and a mounting stand 806, 808. Fig. 28 shows the back cover 800, mounting stand 806, user station assembly 758 mountable to back cover 800 by screws or other fasteners, and a back portion of a telephone handset cradle assembly 812 mountable to the back cover 800 adjacent the station 758. In general, housing portions and components described herein are made of a plastic, such as PC/ABS plastic or similar material.

In the desk mounted configuration of the above-described user stations, microphone 810 is a base mounted directional microphone. The base mounted directional microphone focuses the reception of the user's voice in a conical region directly in front of the station while limiting the reception of other voices and noise outside the conical region. In the wall or vertically mountable configurations of the above-described user stations, an underside mounted microphone is used. The microphone is located on the main user station assembly on the bottom edge (facing downward) underneath the display. The underside mounted microphone allows the station to pick up voices and other sounds around the mounting location. The underside microphone also reduces the likelihood of damage due to user contact and fluids or other contaminants.

In general, the user stations described herein are configurable and scalable, such that features described with reference to one embodiment may be incorporated into other embodiments as well.

## Claims

1. A patient-nurse communication system installable in a healthcare facility having a plurality of locational areas, the plurality of locational areas comprising at least one nursing unit (152, 154, 156) and a plurality of patient rooms (158), the system comprising at least one primary user console (140) and a plurality of secondary consoles (142, 144, 146, 170, 176), wherein each nursing unit (152, 154, 156) includes a primary console and each patient room includes at least one secondary console, the system further comprising wireless location tracking receivers (64) and location tracking badges or tags (66) for locating and tracking of staff members,
the primary console being configured to route or relay calls or alerts to and from other consoles or other components of the system,
each of the plurality of secondary consoles comprising:
a housing (374, 472, 752, 764) defining an interior region, the housing being positionable adjacent a patient location of the healthcare facility,
a communications port coupled to the housing and configured to connect the secondary console to the patient-nurse communication system,
a graphical display (324, 376, 416, 440, 482, 506, 540, 766) supported by the housing,
a memory in the housing comprising first computer program logic and second computer program logic, the first computer program logic being configured to associate the secondary console with a first patient room within the healthcare facility, determine whether calls received from the patient-nurse communication system relate to the first patient room, and format information relating to calls that relate to the first patient room for display on the graphical display,
the second computer program logic being configured to associate the secondary console with a second patient room spaced from the first patient room, determine whether calls from the patient-nurse communication system relate to the second patient room, and format information relating to calls that relate to the second patient room for display on the graphical display, and
electrical circuitry in the housing operable to execute the first computer program logic and the second computer program logic to enable a user to process and manage at the secondary console calls from the patient-nurse communication system that relate to first and second patient rooms,
wherein the system is configured to, upon a call or signal being initiated by one of the secondary consoles:
- locate a nurse or staff member, using the wireless location tracking receivers (64) and location tracking badges or tags (66) for locating and tracking of staff members, and
- route, using the primary console, an appropriate notification to one or more secondary consoles within the closest proximity to the nurse or staff member,
wherein the display of the secondary console is configured to notify or communicate the call or signal initiated by another one of the secondary consoles and routed by the primary console.

2. The system of claim 1, wherein the graphical display (482, 506) of the secondary console comprises a first window and a second window spaced from the first window, the first window is configured to display the information relating to calls that relate to the first patient room and the second window is configured to display the information relating to calls that relate to the second patient room.

3. The system of claim 2, wherein the second patient room has at least one sub-area comprising a patient location and the second window of the graphical display (482, 506) of the secondary console is configured to display information relating to calls that relate to a sub-area of the second patient room.

## Patentansprüche

1. Ein System zur Kommunikation zwischen Patient und Pflegekraft, das in einer medizinischen Einrichtung installiert werden kann, mit einer Vielzahl von Standortbereichen, wobei die Vielzahl von Standortbereichen mindestens eine pflegerische Einheit (152, 154, 156) und eine Vielzahl von Patientenzimmern (158) umfasst, wobei das System mindestens eine übergeordnete Benutzerkonsole (140) und eine Vielzahl von untergeordneten Konsolen (142, 144, 146, 170, 176) umfasst, wobei jede pflegerische Einheit (152, 154, 156) eine übergeordnete Benutzerkonsole beinhaltet und jedes Patientenzimmer mindestens eine untergeordnete Konsole beinhaltet, wobei das System weiter drahtlose Standortverfolgungsempfänger (64) und Standortverfolgungsausweise oder -etiketten (66) zur Ortung und Verfolgung von Mitarbeitern umfasst, wobei die übergeordnete Konsole so konfiguriert ist, dass Rufe oder Alarme an andere oder von anderen Konsolen oder anderen Komponenten des Systems weitergeleitet oder übermittelt werden, wobei die Vielzahl der untergeordneten Konsolen umfasst:
ein Gehäuse (374, 472, 752, 764), das einen Innenbereich definiert, wobei das Gehäuse neben einem Aufenthaltsort eines Patienten der medizinischen Einrichtung positioniert werden kann,
einen Kommunikationsanschluss, der an das Gehäuse gekoppelt und für den Anschluss der untergeordneten Konsole an das System zur Kommunikation zwischen Patient und Pflegekraft konfiguriert ist,
eine grafische Anzeige (324, 376, 416, 440, 482, 506, 540, 766), die von dem Gehäuse getragen wird,
einen Speicher in dem Gehäuse, umfassend eine erste Computerprogrammlogik und eine zweite Computerprogrammlogik, wobei die erste Computerprogrammlogik so konfiguriert ist, dass die untergeordnete Konsole mit einem ersten Patientenzimmer in der medizinischen Einrichtung verbunden wird, um zu bestimmen, ob Rufe vom System zur Kommunikation zwischen Patient und Pflegekraft das erste Patientenzimmer betreffen, und Rufe betreffende Informationen, die das erste Patientenzimmer betreffen, für die Anzeige auf der grafischen Anzeige zu formatieren,
wobei die zweite Computerprogrammlogik so konfiguriert ist, dass die untergeordnete Konsole mit einem zweiten Patientenzimmer im Abstand von dem ersten Patientenzimmer verbunden wird, um zu bestimmen, ob Rufe vom System zur Kommunikation zwischen Patient und Pflegekraft das zweite Patientenzimmer betreffen, und Rufe betreffende Informationen, die das zweite Patientenzimmer betreffen, für die Anzeige auf der grafischen Anzeige zu formatieren, und
elektrische Schaltkreise im Gehäuse, die zur Ausführung der ersten Computerprogrammlogik und der zweiten Computerprogrammlogik bedienbar sind, um einem Benutzer die Verarbeitung und Verwaltung der Rufe vom System zur Kommunikation zwischen Patient und Pflegekraft, die das erste und zweite Patientenzimmer betreffen, an der untergeordneten Konsole zu ermöglichen,
wobei das System so konfiguriert ist, dass nach einem von einer der untergeordneten Konsolen ausgelösten Ruf oder Signal:
- eine Pflegekraft oder ein Mitarbeiter mithilfe der drahtlosen Standortverfolgungsempfänger (64) und Standortverfolgungsausweise oder -etiketten (66) zur Ortung und Verfolgung von Mitarbeitern geortet wird, und
- mithilfe der übergeordneten Konsole eine entsprechende Mitteilung an eine oder mehrere untergeordnete Konsolen in unmittelbarer Nähe der Pflegekraft oder des Mitarbeiters weitergeleitet wird,
wobei die Anzeige der untergeordneten Konsole so konfiguriert ist, dass der Ruf oder das Signal, der bzw. das durch eine andere der untergeordneten Konsolen ausgelöst wird, gemeldet oder kommuniziert wird und von der übergeordneten Konsole weitergeleitet wird.

2. Das System nach Anspruch 1, wobei die grafische Anzeige (482, 506) der untergeordneten Konsole ein erstes Fenster und ein zweites Fenster im Abstand von dem ersten Fenster umfasst, wobei das erste Fenster so konfiguriert ist, dass die Rufe betreffenden Informationen, die das erste Patientenzimmer betreffen, angezeigt werden, und das zweite Fenster so konfiguriert ist, dass die Rufe betreffenden Informationen, die das zweite Patientenzimmer betreffen, angezeigt werden.

3. Das System nach Anspruch 2, wobei das zweite Patientenzimmer mindestens einen Unterbereich aufweist, der einen Aufenthaltsort eines Patienten umfasst, und das zweite Fenster der grafischen Anzeige (482, 506) der untergeordneten Konsole so konfiguriert ist, dass Rufe betreffende Informationen angezeigt werden, die den Unterbereich des zweiten Patientenzimmers betreffen.

## Revendications

1. Un système de communication patient-infirmière pouvant être installé dans un établissement de santé ayant une pluralité de zones d'emplacement, la pluralité de zones d'emplacement comprenant au moins une unité de soins infirmiers (152, 154, 156) et une pluralité de chambres de patient (158), le système comprenant au moins une console d'utilisateur principale (140) et une pluralité de consoles secondaires (142, 144, 146, 170, 176), dans lequel chaque unité de soins infirmiers (152, 154, 156) comprend une console principale et chaque chambre de patient comprend au moins une console secondaire, le système comprenant également des récepteurs de suivi de localisation sans fil (64) et des badges ou des étiquettes de suivi de localisation (66) pour la localisation et le suivi des membres du personnel, la console principale étant configurée pour acheminer et relayer les appels ou les alertes vers et en provenance d'autres consoles ou autres éléments du système, chacune de la pluralité des consoles secondaires comprenant :
un boîtier (374, 472, 752, 764) définissant une région intérieure, le boîtier pouvant être placé à côté d'un emplacement de patient de l'établissement de soins,
un port de communication couplé au boîtier et configuré pour connecter la console secondaire à un système de communication patient-infirmière,
un écran graphique (324,376,416,440,482,506,540, 766) supporté par le boîtier,
une mémoire dans le boîtier comprenant une première logique de programme informatique et une deuxième logique de programme informatique, la première logique de programme informatique étant configurée pour associer la console secondaire à une première chambre de patient au sein de l'établissement de soins, pour déterminer si les appels reçus en provenance du système de communication patient-infirmière sont liés à la première chambre de patient, et les informations de format relatives aux appels liés à la première chambre de patient pour affichage sur l'écran graphique,
la deuxième logique de programme informatique étant configurée pour associer la console secondaire à une deuxième chambre de patient éloignée de la première chambre de patient, pour déterminer si les appels en provenance du système de communication patient-infirmière sont liés à la seconde chambre de patient, et les informations de format relatives aux appels liés à la seconde chambre de patient pour affichage sur l'écran graphique, et
un circuit électrique dans le boîtier fonctionnant pour exécuter la première logique du programme informatique et la deuxième logique du programme informatique pour permettre à un utilisateur de traiter et de gérer sur la console secondaire les appels en provenance du système de communication patient-infirmière liés à la première et à la deuxième chambre de patient,
dans lequel le système est configuré pour, sur un appel ou un signal étant initié par l'une des consoles secondaires :
- localiser une infirmière ou un membre du personnel, en utilisant des récepteurs de suivi de localisation sans fil (64) et des badges ou des étiquettes de suivi de localisation (66) pour la localisation et le suivi des membres du personnel, et
- acheminer, en utilisant la console principale, une notification appropriée jusqu'à une ou plusieurs consoles secondaires au plus près de l'infirmière ou du membre du personnel,
dans lequel l'écran de la console secondaire est configuré pour notifier ou communiquer l'appel ou le signal initié par une autre des consoles secondaires et acheminé par la console principale.

2. Le système selon la revendication 1, dans lequel l'écran graphique (482, 506) de la console secondaire comprend une première fenêtre et une seconde fenêtre espacée de la première fenêtre, la première fenêtre est configurée pour afficher les informations relatives aux appels liés à la première chambre de patient et la deuxième fenêtre est configurée pour afficher les informations relatives aux appels liés à la seconde chambre de patient.

3. Le système selon la revendication 2, dans lequel la deuxième chambre de patient a au moins une sous-région comprenant une localisation de patient et la deuxième fenêtre de l'écran graphique (482, 506) de la console secondaire est configurée pour afficher des informations relatives aux appels liés à une sous-région de la deuxième chambre de patient.
